# EUROPEAN PATENT APPLICATION

(11) **EP 4 631 510 A1**
(43) Date of publication of application: **15.10.2025**
(21) Application number: 24170001.2
(22) Date of filing: 12.04.2024
(51) Int. Cl.: A61K 31/727, A61P 31/00, A61P 37/00, A61P 43/00

(54) **LOW ANTICOAGULANT HEPARIN FOR USE IN THE PREVENTION AND TREATMENT OF SEPSIS**

(71) Applicant: Matisse Pharmaceuticals B.V., 6163 JT Geleen (NL); Stichting Amsterdam UMC, 1081 HV Amsterdam (NL)
(72) Inventor: EKHART, Peter Frank, GELEEN (NL); SCHRIJVER, Roy Louis Johannes, GELEEN (NL); VAN LEEUWEN, Simone Johanna Maria, GELEEN (NL); VAN MOURIK, Niels, AMSTERDAM (NL); MÜLLER, Marcella Catharina Antoinetta, AMSTERDAM (NL); VAN DER POLL, Tom, AMSTERDAM (NL); VLAAR, Alexander Petrus Johannes, AMSTERDAM (NL); VAN VUGHT, Lonneke Alette, AMSTERDAM (NL)
(74) Representative: Algemeen Octrooi- en Merkenbureau B.V.

(57) **Abstract**

This invention is in the field of medical treatment, in particular the invention provides a method for the prevention and treatment for sepsis or septic shock. In particular the invention provides dosages and formulation for low anticoagulant heparin in the treatment of sepsis or septic shock. The invention further provides compositions for use in reducing C-reactive protein (CRP) levels in a patient, for example in the treatment, prevention or amelioration of disease where CRP plays a role, such as sepsis.

## Description

### Field of the invention

This invention is in the field of medical treatment, in particular the invention provides a method for the prevention and treatment for sepsis or septic shock. In particular the invention provides dosages and formulations for low anticoagulant heparin in the treatment of sepsis or septic shock. The invention further provides compositions for use in reducing C-reactive protein (CRP) levels in a patient, for example in the treatment, prevention or amelioration of disease where CRP plays a role, such as sepsis.

### Background of the invention

Sepsis is defined as life-threatening organ dysfunction caused by a dysregulated host response to infection. The immune system may develop an inflammatory response to microbes in the blood, urine, lungs, skin, or other tissues. A lay term for sepsis is blood poisoning, more aptly applied to septicemia, below..

Sepsis is usually treated in the intensive care unit with intravenous fluids and antiinfectives. If fluid replacement is insufficient to maintain blood pressure, specific vasopressor medications can be used. Mechanical ventilation and dialysis may be needed to support the function of the lungs and kidneys, respectively..

Septic shock is a subset of sepsis in which underlying circulatory and cellular/ metabolic abnormalities are profound enough to substantially increase mortality.

The process of infection by bacteria or fungi can result in systemic signs and symptoms that are variously described. Approximately 70% of septic shock cases were once traceable to Gram staining gram-negative bacilli that produce endotoxins; however, with the emergence of MRSA and the increased use of arterial and venous catheters, Gram-positive cocci are implicated approximately as commonly as bacilli. In rough order of increasing severity, these are bacteremia or fungemic; sepsis, septic shock; multiple organ dysfunction syndrome, and death.

Despite the manifold of different therapies, there exists a need in the art for reliable, safe, affordable and easily applicable therapies for sepsis and related disorders as presented above.

The present invention addresses the above shortcomings, problems and concerns in that it provides a method for the prevention and/or treatment of sepsis, SIRS, septic shock.

### Summary of the invention

In a first aspect the invention relates to low anticoagulant heparin for use in the treatment or prevention of sepsis, SIRS or septic shock in a subject, wherein the low anticoagulant heparin is administered to the subject in a dose of at least 0.15 mg/(kg*hr) body weight of the subject.

In a second aspect the invention relates to low anticoagulant heparin for use in the treatment or prevention of a disease, the use comprising reducing C-reactive protein (CRP) plasma levels in the patient.

### Brief description of the figures

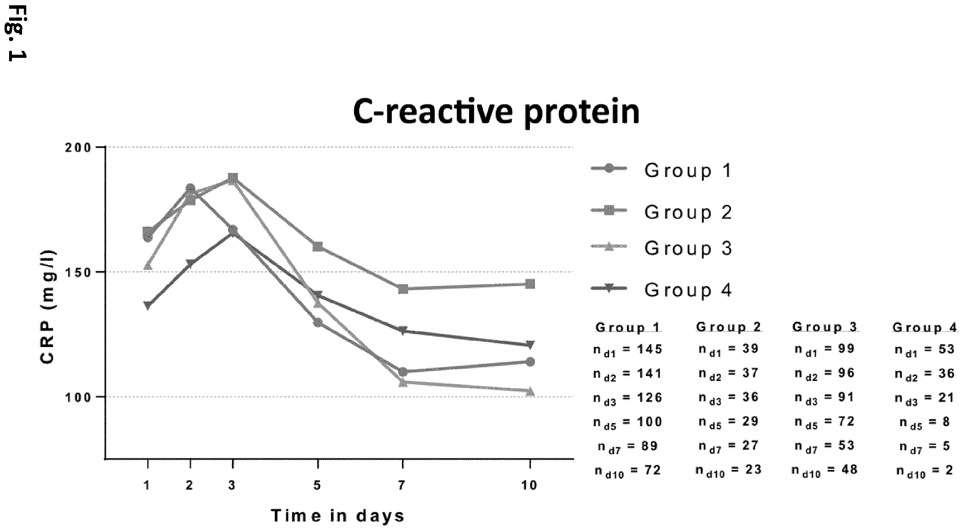

Fig. 1 depicts dynamic changes of CRP after standard of care treatment within prespecified subgroups: (group 1) patients with initial sepsis without progression to septic shock; (group 2) patients with initial sepsis with progression to septic shock; (group 3) patients with initial septic shock with clinical improvement to sepsis without shock; (group 4) patients with initial septic shock without clinical improvement to sepsis without shock.

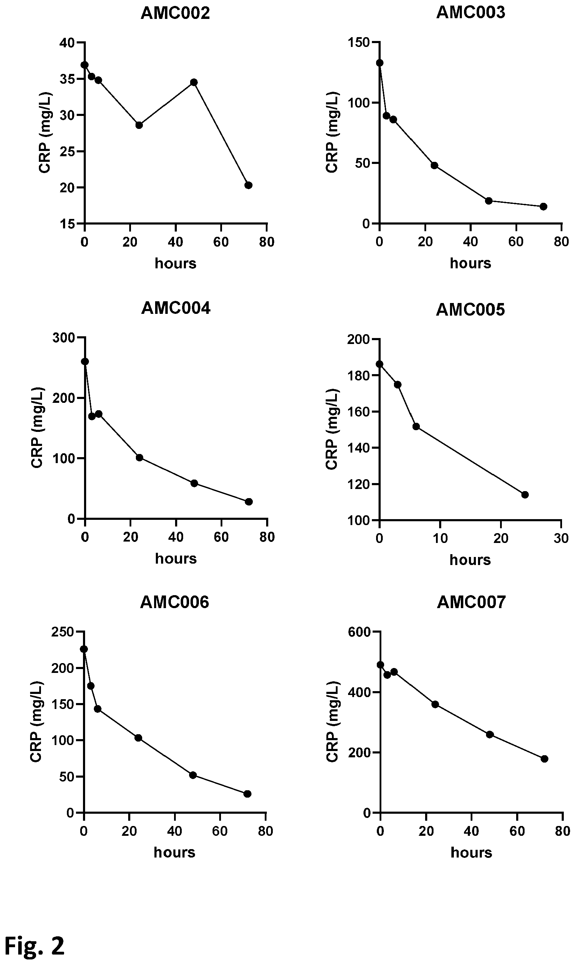

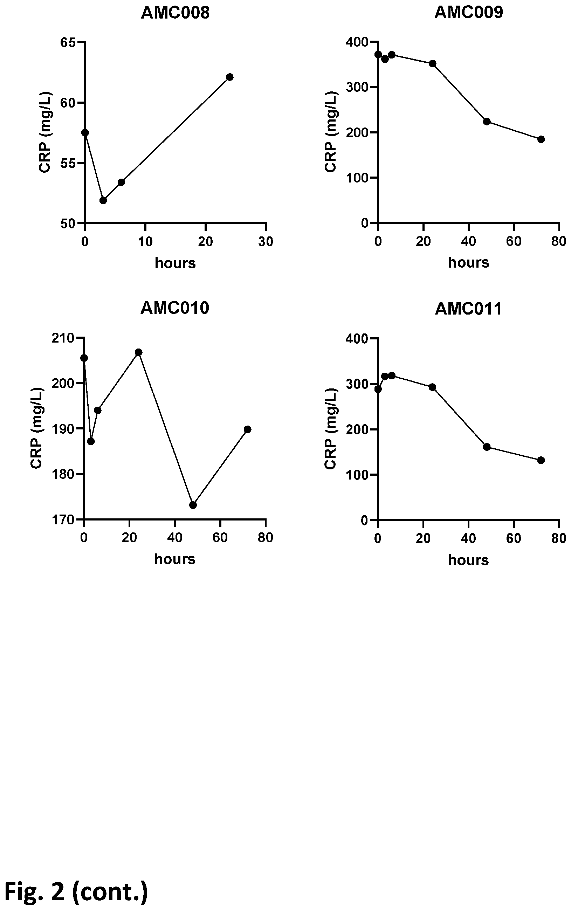

Fig. 2 depicts CRP levels and individual patients over time. CRP levels are indicated in mg/ml, measurements were made at 0, 3, 6, 24, 48 and 72 hours.

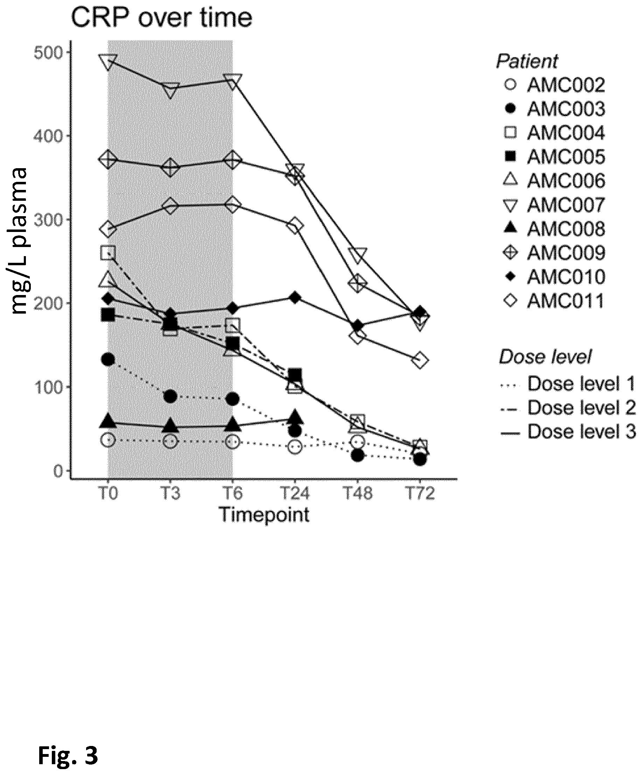

Fig. 3 depicts an overlay of the CRP levels of all ten patients; timepoints are not to scale. The grey area depicts the time of intravenous administration of low anticoagulant heparin. Dose levels correspond to: Dose level 1: 0.15 mg/kgh (patents 002-003); Dose level 2: 0.45 mg/kgh (patients 004-005); Dose level 3: 0.9 mg/kgh (patients 006-011).

### Definitions

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. For example, a method for administrating a pharmaceutical agent includes the administrating of a plurality of molecules (e.g., 10's, 100's, 1000's, 10's of thousands, 100's of thousands, millions, or more molecules).

As used herein, "about" and "approximately", when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed invention. Unless otherwise clear from context, all numerical values provided herein include numerical values modified by the term "about."

As used herein, "and/or" refers to a situation wherein one or more of the stated cases may occur, alone or in combination with at least one of the stated cases, up to with all of the stated cases.

As used herein, "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

As used herein, "conventional techniques" or "methods known to the skilled person" refer to a situation wherein the methods of carrying out the conventional techniques used in methods of the invention will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, "exemplary" or "for example" means "serving as an example, instance, or illustration," and should not be construed as excluding other configurations, including those disclosed herein.

Throughout this disclosure, various aspects of the invention can be presented in a range format. It should be understood that the description in range format is merely for convenience and should not be construed as a limitation on the scope of the invention. The description of a range should be considered to have specifically disclosed all the possible subranges as well as individual numerical values within that range including both integers and non-integers. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, 6 etc. This applies regardless of the breadth of the range.

As used herein, the term "pharmaceutical composition" refers to a composition formulated in pharmaceutically acceptable or physiologically-acceptable compositions for administration to a cell or subject. The compositions of the invention may be administered in combination with other agents as well, provided that the additional agents do not adversely affect the ability of the composition to deliver the intended therapy. The pharmaceutical composition often comprise, in addition to a pharmaceutical active agent, one or more pharmaceutical acceptable carriers (or excipients).

As used herein, "treatment", "treating", "palliating", "alleviating" and "ameliorating" in the context of a subject to be treated, all refer to an approach for obtaining beneficial or desired results including, but not limited to, therapeutic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. As used herein, "prevention" and "preventing" refers to an approach for reducing in part or in full the change of developing adverse effects, for example normally associated with the use of a particular drug or agent. Within the context of the current invention, for example, the terms may refer to preventing, treating or reducing the effects of a neurological disorder.

As used herein, the term "low-anticoagulant heparin" intends to refer to heparins can be obtained either by removing chains containing the antithrombin-binding sequence, or by inactivating critical functional groups or units of this sequence. The term low-anticoagulant heparin also refers to non-anticoagulant heparin, and includes regioselectively desulfated heparins, 'glycol-split' heparins and pentasaccharide depleted heparin. Thus the terms "low anticoagulant heparin" and "pentasaccharide depleted heparin" further include chemically modified heparin such as but not limited to pentasaccharide depleted heparin modified with periodate oxidation, N-acetylation, N-deacetylation, N-sulfation, O-sulfation, 2-O desulfation, and complete desulfation as for example described in Raman et al., hereby incorporated by reference in its entirety.

### Detailed description of the invention

The invention is defined herein, and in particular in the accompanying claims. Subject-matter which is not encompassed by the scope of the claims does not form part of the present claimed invention.

It is contemplated that any method, use or composition described herein can be implemented with respect to any other method, use or composition described herein. Embodiments or preferences discussed in the context of methods, use and/or compositions of the invention may likewise be employed with respect to any other method, use or composition described herein. Thus, an embodiment or preference pertaining to one method, use or composition may be applied to other methods, uses and compositions of the invention as well.

Any references in the description to methods of treatment refer to the compounds, pharmaceutical compositions and medicaments of the present invention for use in a method for treatment of the human (or animal) body by therapy.

The present invention broadly relates to the use of low anticoagulant heparin in the treatment of diseases like sepsis by reducing CRP.

Heparin is a first-line anticoagulant and has been used for over a century. It is a heterogeneous, linear, highly sulfated, anionic glycosaminoglycan with a broad distribution in relative molecular weight and charge density. These structural properties allow heparin to selectively interact with multiple proteins, leading to heparin's various pharmacological functions, such as anticoagulant, anti-viral, antitumor and anti-inflammatory activities (Wang et al.).

Heparin is a mixture of polysaccharide chains (Casu et al. 1989). "Structure of heparin and heparin fragments." Ann N Y Acad Sci 556: 1-17). The composition of the polysaccharide chains and their length varies. Chains with a so-called pentasaccharide domain bind strongly to anti-thrombin (AT), which is one of the major circulating anticoagulant proteins (Casu et al. 1981).

Heparin with a decreased anti-coagulant activity is known in the art and there are several methods known for its preparation. For example, heparin may be depleted from its pentasaccharides by affinity chromatography, thereby obtaining non-anticoagulant heparin or heparin with a decreased anti-coagulant activity. Heparin may also be chemically treated in order to obtain heparin with a decreased anti-coagulant activity included but not limited to regioselectively desulfated heparins, and 'glycol-split' heparins like for example heparin obtained by periodate oxidation followed by borohydride reduction. Thus when used herein the term low anticoagulant heparin intends to refer to any heparin that is modified to reduce its anticoagulant properties. Non limiting examples are pentasaccharides depleted heparin, regioselectively desulfated heparins and glycol-split heparins like heparin obtained by periodate oxidation followed by borohydride reduction.

Pentasaccharide depleted heparin may be obtained from unfractionated heparin (UFH) by methods known in the art, for example described in WO2013007771A1 or WO2016198578A1 (each herein incorporated by reference in its entirety). In a preferred method, the pentasaccharide depleted heparin is obtained by affinity chromatography. Therein, UFH is passed through a column that contains immobilized AT. The molecules that contain the pentasaccharide sequence bind to the column, whereas other material passes. Unbound material is called Low Affinity Material (LAM), whereas material that does bind is called High Affinity Material (HAM). LAM is substantially reduced in pentasaccharides and subsequently in its anticoagulant activity, whereas HAM has an elevated anticoagulant activity.

LAM may also be described as the pentasaccharide-depleted fraction of natural heparin. The terms pentasaccharide depleted heparin, LAM and M6229 are used interchangeably herein.

The term pentasaccharide-depleted heparin in this context is used to refer to a fraction of heparin wherein the content of pentasaccharides is substantially reduced in comparison to commercially available heparin.

The term substantially reduced or decreased as used herein means reduced with at least 10%, such as 20% or 30%, more preferably 40 or 50%, even more preferred, more than 60% or 70% or 80% such as 90% or more than 98% such as more than 99% or even 100%. It is most preferred when the pentasaccharide depleted fraction does not contain any detectable pentasaccharides when tested for thrombin generation as described by Hemker et al., (2003) infra. Conversely, heparin with a decreased anti-coagulant activity means heparin with a reduced anti-coagulant activity, such as reduced with at least 10%, such as 20% or 30%, more preferably 40 or 50%, even more preferred, more than 60% or 70% or 80% such as 90% or more than 98% such as more than 99% or even 100%. It is most preferred when the heparin with a decreased anti-coagulant activity does not contain any detectable anti-coagulant activity when tested for thrombin generation as described by Hemker et al., (2003) infra. In the experimental section it is described in detail how a pentasaccharide-depleted heparin may be obtained. It is called LAM therein, abbreviation of Low Affinity Material.

C-reactive protein (CRP) is one of the major members of the family of acute phase proteins (APP). Interest in this CRP was the result of a seminal discovery of its pattern of response to pneumococcal infection in humans. CRP has the unique property of reacting with phosphocholine-containing substances, such as pneumococcal C-polysaccharide, in the presence of Ca²⁺ (Bhattacharya et al.).

CRP is produced in the liver. Plasma concentrations are normally under 5 mg/l but increase several fold after sepsis, trauma, inflammation and other stimuli that involve tissue damage. Bacterial infection is also a potent stimulus, leading to a rapid elevation of CRP levels within hours. Interleukin 6 (IL-6) is thought to be the main mediator stimulating CRP production, but other cytokines like IL-1 and tumor necrosis factor are also involved. Changes of plasma CRP levels can be useful in the diagnosis of infection and in the follow-up of the clinical course, with a fall indicating resolution of infection (Povoa et al.). Although CRP levels have not been found useful for diagnostic and prognostic purposes, in patients with cardiogenic shock, increasing CRP levels have recently been shown to indicate increased short-term mortality (Schupp et al.). Thus reducing plasma CRP levels is anticipated to be an useful outcome of a therapy. No treatments are currently known to reduce CRP levels that are suitable for treatment of sepsis.

The present invention broadly describes that low anticoagulant heparin can be used in the treatment of sepsis or other disease by lowering the circulating CRP levels in a patient. The inventors describe herein data describing a first in human trial of low anticoagulant heparin in septic patients. The result unexpectedly demonstrate that low anticoagulant heparin lowers CRP values in septic patients to levels of 50% or even lower within a time span of 72 hours or even less. To put this in perspective, using standard of care treatment results in a comparable reduction of CRP in septic patients in a time span of 5 to 7 days, indicating a strong improvement over standard of care treatment.

Sepsis is a life-threatening organ dysfunction caused by a dysregulated host response to infection. Septic shock is a subset of sepsis in which particularly profound circulatory, cellular, and metabolic abnormalities are associated with a greater risk of mortality than with sepsis alone. Definitions for sepsis and septic shock have been updated and described in Singer et al., which is incorporated by reference in its entirety. When used herein preventing sepsis, SIRS or septic shock may for example refer to treatment of a subject at risk of developing sepsis, SIRS or septic shock. For example a subject may be at risk for developing sepsis, SIRS or septic shock when the subject has an infection or a chronic disease.

Therefore, in a first aspect the invention relates to low anticoagulant heparin for use in the treatment or prevention of sepsis, SIRS or septic shock in a subject, wherein the low anticoagulant heparin is administered to the subject in a dose of at least 0.15 mg/(kg*hr) body weight of the subject. In an alternative aspect the invention relates to a method of treating or preventing sepsis in a subject in need thereof, the method comprising administering low anticoagulant heparin is administered to the subject in a dose of at least 0.15 mg/(kg*hr) body weight of the subject.

The inventors herein show in vivo data of septic patients using three different dosages. (0.15 mg/(kg*hr) body weight, 0.45 mg/(kg*hr) body weight and 0.9 mg/(kg*hr) body weight; each administered for 6 hours). Thus in an embodiment the low anticoagulant heparin is administered to the subject in a dose of at least 0.45 mg/(kg*hr) body weight of the subject. In an embodiment the low anticoagulant heparin is administered to the subject in a dose of at least 0.9 mg/(kg*hr) body weight of the subject. Thus in an embodiment the low anticoagulant heparin administration is provided in a dose of at least 0.15 mg/(kg*hr) body weight of the subject, such as for example 0.15, 0.20, 0.25, 0.30, 0.35, 0.40, 0.45, 0.50, 0.55, 0.60, 0.65, 0.70, 0.75, 0.80, 0.85, 0.90, 0.95, or 1.0 mg/(kg*hr) body weight of the subject or more. In an embodiment the low anticoagulant heparin administration is provided in a dose of 2.0 mg/(kg*hr) body weight of the subject or less, such as for example 2.0, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, or 1.0 mg/(kg*hr) body weight of the subject or less. Thus in a further embodiment the low anticoagulant heparin administration is provided in a dose of between 0.15 and 2.0 mg/(kg*hr) body weight of the subject, preferably between 0.20 and 1.5, more preferably between 0.25 and 1.0 mg/(kg*hr) body weight of the subject. The dosage unit mg/(kg*hr) refers to the total amount of compound in mg administered in one hour per kg body weight of the subject. The administration may be continuous (e.g. intravenously) or by one or more single administrations (e.g. injections). In the latter case it is understood that the compound may be provided less than once per hour and the unit mg/(kg*hr) refers to the average administered dose in mg per kg body weight of the subject per hour over the total time of treatment. For example this may be the case when the low anticoagulant heparin is administered subcutaneously, e.g. by daily subcutaneous injection.

Although heparin has been suggested as a potential treatment for sepsis, so far no successful treatment has been reported, as for example evidenced by Jaimes et al. who report that unfractionated suggested that unfractionated heparin (UFH) may be a feasible and safe intervention in sepsis, however their study was not able to demonstrate a beneficial effect on the chosen primary outcomes or in the 28-day mortality rate. Without wishing to be bound by theory, it is postulated that heparin based therapies are not successful due to anticoagulant properties of heparin. The maximum dose of heparin that can be used is typically around 0.1 mg/(kg*hr) body weight of the patient. Due to these anticoagulant properties higher doses of heparin cannot be used in septic patients, among others, as the anticoagulant properties cause bleeding and may be fatal. Because the herein described low anticoagulant heparin has low anticoagulant properties, a higher dose can be administered which surprisingly results in quick reduction of CRP levels in the patient when exceeding the safe level of unfractionated heparin (e.g. dose levels of 0.15, 0.45 or even 0.9 mg/(kg*hr)). WO2013007771A1 suggest the use of pentasaccharide depleted heparin in the treatment of sepsis, however does not disclose any dosages, let alone which dosages can be safely used or above which dosage CRP is effectively reduced in patients. The present invention provides the surprising finding that contrary to unfractionated heparin, low anticoagulant heparin can be safely administered at higher doses and more importantly results in the quick reduction of plasma CRP levels at such high doses.

In the present clinical trial pentasaccharide-depleted heparin was administered continuously for six hours, however it is understood that shorter or longer administration times may be used. It is further envisioned that the low anticoagulant heparin administration is provided for a much longer period as currently tested., e.g. 72 hours, 120 hours or even longer. Therefore in an embodiment the low anticoagulant heparin is administered over a period of at least 2 hours, preferably at least 4 hours more preferably at least 5 hours most preferably about 6 hours or longer. Thus for example the low anticoagulant heparin administration is provided for about 4 to 12 hours, preferably 5 to 8 hours, such as for example 4, 4.5, 5, 5.5, 6, 6.5, 7, 8, 9, 10, 11, or 12 hours. Alternatively the low anticoagulant heparin administration is provided for 48 hours or longer, for example at least 60, preferably at least 72, more preferably at least 96 most preferably at least 120 hours or even longer. In a further preferred embodiment the low anticoagulant heparin administration is provided for 48, 50, 52, 54, 56, 58, 60, 62, 64, 66, 68, 70, 72, 74, 76, 78, 80, 82, 84, 86, 88, 90, 92, 94, 96, 98, 100, 104, 108, 112, 116, 120, 124, 128, 132, 136, 140 hours or longer.

It is further envisioned that the same absolute amount of low anticoagulant heparin is administered over a shorter or longer period of time, for example by one or more bolus subcutaneous injections or by continuous intravenous administration or one or more bolus intravenous injections. Therefore in an embodiment the low anticoagulant heparin is administered to the subject in a dose of at least 0.90 mg/kg body weight of the subject, preferably at least 2.7 mg/kg body weight of the subject, more preferably at least 5.4 mg/kg body weight of the subject. In an embodiment the low anticoagulant heparin is administered to the subject in a dose of between 0.9 and 10 mg/kg body weight of the subject, preferably between 1.0 and 8 mg/kg body weight of the subject, more preferably between 1.2 and 6 mg/kg body weight of the subject. In an embodiment the low anticoagulant heparin is provided in a dose of 0.9, 1.0, 1.1, 1.2, 1.3, 1.4, 1.5, 1.6, 1.7, 1.8, 1.9, 2.0, 2.2, 2.4, 2.6, 2.8, 3.0, 3.2, 3.4, 3.6, 3.8, 4.0, 4.5, 5.0, 5.0, 5.5, 6.0, 6.5, 7.0, 7.5, 8.0, 8.5, 9.0, 9.5, or 10 mg/kg body weight of the subject.

The present data is obtained with intravenous infusion in patients, however the invention should not be construed as limited to such mode of administration. It is anticipated that any form of parenteral administration is suitable, including intravenous, subcutaneous, intramuscular or intradermal administration. In an embodiment the low anticoagulant heparin is administered parenterally. In an embodiment the low anticoagulant heparin is administered intravenously or subcutaneously. The term intravenous administration encompasses bolus administration which may be administered as an IV push, administration through an IV infusion and secondary administration. When used herein secondary administration refers to medication to be administered intravenously at the same time as an infusion may be connected to the primary tubing, and is also referred to as a secondary IV, or IV piggyback.

Thus for example in case the low anticoagulant heparin is administered by subcutaneous injection, a dose of 0.15 mg/(kg*hr) body weight of the subject may for example correspond to a single subcutaneous injection of 0.15 mg/kg body weight of the subject each hour, or an injection of 0.30 mg/kg body weight of the subject each two hours, or an injection of 0.45 mg/kg body weight of the subject each three hours, etc. The dose can be scaled accordingly in case a higher dosage regime is used, e.g. 0.45 or 0.9 mg/(kg*hr). Thus, in case the low anticoagulant heparin is administered by subcutaneous injection, a dose of 0.45 mg/(kg*hr) body weight of the subject may for example correspond to a single subcutaneous injection of 0.45 mg/kg body weight of the subject each hour, or an injection of 0.90 mg/kg body weight of the subject each two hours, or an injection of 1.35 mg/kg body weight of the subject each three hours, etc. In case the low anticoagulant heparin is administered by subcutaneous injection, a dose of 0.90 mg/(kg*hr) body weight of the subject may for example correspond to a single subcutaneous injection of 0.90 mg/kg body weight of the subject each hour, or an injection of 1.8 mg/kg body weight of the subject each two hours, or an injection of 2.7 mg/kg body weight of the subject each three hours, etc.

CRP baseline levels in plasma are generally well below 2 mg/l, but are found to increase dramatically in sepsis to levels above 50 mg/l. Normal ranges of plasma CRP are generally in the range of 0.3 to 1.0 mg/L plasma, so values above 2.5 mg CRP / L plasma (such as 3 mg CRP / L plasma) can be regarded as abnormally high. Using standard of care treatment CRP levels typically take over 10 days to return to levels below 100 mg/I, and equally long to reduce to 50% of the peak value which was reached around day 3 of sepsis. The data reported here shows that administration of low anticoagulant heparin avoids CRP to peak at day three but instead stabilizes CRP levels and in most cases result in rapid decline of CRP values.

Thus in an embodiment the low anticoagulant heparin is administered to a subject when plasma CRP levels exceed 3 mg/L, preferably when the CRP levels exceed 4, 5, 6, 7, 8, 9, 10, 15, 20, 25, 30, 40, 50, 60, 70, 80, 90 or 100 mg/L in plasma of the subject. In an embodiment the use comprises reducing the amount of C reactive protein (CRP) in the subject. In an embodiment the use comprises avoiding an increase in CRP levels. In an embodiment the invention further envisions monitoring the plasma CRP values in order to decide if low anticoagulant heparin administration should continue. Therefore in an embodiment the low anticoagulant heparin is administered to a subject as long as the plasma CRP levels exceed a threshold value. For example the threshold value may be 3, 4, 5, 6, 7, 8, 9, 10, 15, 20, 25, 30, 35, 40, 45, or 50 mg CRP / L plasma. In an embodiment the level of CRP is reduced to 50% or less in 72 hours. For example the CRP levels have been reduced to levels of 50% compared to CRP levels at diagnosis at 72 hours, preferably 45%, more preferably 40%, 35%, 30%, or 25% or lower. Alternatively the CRP levels have been reduced to levels of 50% compared to CRP levels at diagnosis at 72 hours post diagnosis, preferably at 66 hours, 60 hours, 54 hours, 48 hours, or earlier.

Therefore in a further embodiment the invention relates to low anticoagulant heparin for use in the treatment or prevention of sepsis, SIRS or septic shock in a subject, wherein the use comprises monitoring the subject and administering the low anticoagulant heparin to the subject when the low anticoagulant heparin is administered to the subject plasma CRP levels exceed 3 mg/L, preferably when the CRP levels exceed 3, 4, 5, 6, 7, 8, 9, 10, 15, 20, 25, 30, 40, 50, 60, 70, 80, 90 or 100 mg/L in plasma of the subject, wherein the low anticoagulant heparin is administered in a dose of at least 0.15 mg/(kg*hr) body weight of the subject. In a further embodiment the invention relates to low anticoagulant heparin for use in the treatment or prevention of sepsis, SIRS or septic shock in a subject, wherein the use comprises, wherein the use comprises administering the low anticoagulant heparin in a dose of at least 0.15 mg/(kg*hr) body weight of the subject and subsequent monitoring the subject, wherein the low anticoagulant heparin is administered to the subject until the subject plasma CRP levels fall below 50 mg/L, preferably until the CRP levels fall below 40, 30, 25, 20, 15, 10, 9, 8, 7, 6, 5, 4, or 3 mg/L in plasma of the subject.

In an embodiment the subject is a human or a non-human mammal. Preferably the subject is human. In an embodiment the low anticoagulant heparin is pentasaccharide depleted heparin.

It has been described that CRP plays a role in many disorders, such as sepsis, SIRS, septic shock, inflammatory bowel disease (IBD; Folwaczny et al., Ang et al.), Chronic obstructive pulmonary disease (COPD; Ashoor et al.), pancreatitis (Qiu et al, Lu et al., Jin et al.), abdominal aortic aneurism, atherosclerosis, (rheumatoid) arthritis, cardiovascular disease, type 2 diabetes mellitus, obesity, age related macular degeneration, haemorrhagic stroke, Alzheimer's disease, and Parkinson's disease (Battacharya et al.). As the inventors herein convincingly demonstrate that CRP levels can be quickly reduced following low anticoagulant heparin administration it is anticipated and made plausible that low anticoagulant heparin can be successfully used in other disorder wherein CRP plays a role, buy reducing circulating CRP levels.

Therefore, in a second aspect the invention relates to low anticoagulant heparin for use in the treatment or prevention of a disease, the use comprising reducing C-reactive protein (CRP) in the patient. Alternatively the invention provides a method of treating or preventing a disease in a subject in need thereof, the method comprising administering low anticoagulant heparin to subject in need thereof, thereby reducing C-reactive protein (CRP) in the patient. In an embodiment the disease is selected from sepsis, SIRS, septic shock, inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, age related macular degeneration, haemorrhagic stroke, Alzheimer's disease, and Parkinson's disease.

It is anticipated that for other indications CRP levels could be reduced using a similar dose as is presented for sepsis. Therefore, in an embodiment the low anticoagulant heparin may be administered to the subject in a dose of at least 0.15 mg/(kg*hr) body weight of the subject. In an embodiment the low anticoagulant heparin is administered to the subject in a dose of at least 0.45 mg/(kg*hr) body weight of the subject. In an embodiment the low anticoagulant heparin is administered to the subject in a dose of at least 0.9 mg/(kg*hr) body weight of the subject. Thus in an embodiment the low anticoagulant heparin administration is provided in a dose of at least 0.15 mg/(kg*hr) body weight of the subject, such as for example 0.15, 0.20, 0.25, 0.30, 0.35, 0.40, 0.45, 0.50, 0.55, 0.60, 0.65, 0.70, 0.75, 0.80, 0.85, 0.90, 0.95, or 1.0 mg/(kg*hr) body weight of the subject. In an embodiment the low anticoagulant heparin administration is provided in a dose of at most 2.0 mg/(kg*hr) body weight of the subject, such as for example 2.0, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, or 1.0 mg/(kg*hr) body weight of the subject. Thus in a further embodiment the low anticoagulant heparin administration is provided in a dose of between 0.15 and 2.0 mg/(kg*hr) body weight of the subject, preferably between 0.20 and 1.5, more preferably between 0.25 and 1.0 mg/(kg*hr) body weight of the subject. However it is appreciated that in other disorders CRP levels may increase more, or less, when comparing to sepsis, so the dose may be adjusted accordingly.

In an embodiment the low anticoagulant heparin is administered over a period of at least 2 hours, preferably at least 4 hours more preferably at least 5 hours most preferably about 6 hours or longer. In an embodiment the low anticoagulant heparin provided for a much longer period as currently tested., e.g. 72 hours, 120 hours or even longer. Therefore in an embodiment the low anticoagulant heparin is administered over a period of at least 2 hours, preferably at least 4 hours more preferably at least 5 hours most preferably about 6 hours or longer. Thus for example the low anticoagulant heparin administration is provided for about 4 to 12 hours, preferably 5 to 8 hours, such as for example 4, 4.5, 5, 5.5, 6, 6.5, 7, 8, 9, 10, 11, or 12 hours. Alternatively the low anticoagulant heparin administration is provided for 48 hours or longer, for example at least 60, preferably at least 72, more preferably at least 96 most preferably at least 120 hours or even longer. In a further preferred embodiment the low anticoagulant heparin administration is provided for 48, 50, 52, 54, 56, 58, 60, 62, 64, 66, 68, 70, 72, 74, 76, 78, 80, 82, 84, 86, 88, 90, 92, 94, 96, 98, 100, 104, 108, 112, 116, 120, 124, 128, 132, 136, 140 hours or longer.

In an embodiment the low anticoagulant heparin is administered to the subject in a dose of at least 0.90 mg/kg body weight of the subject, preferably at least 2.7 mg/kg body weight of the subject, more preferably at least 5.4 mg/kg body weight of the subject. In an embodiment the low anticoagulant heparin is administered to the subject in a dose of between 0.9 and 10 mg/kg body weight of the subject, preferably between 1.0 and 8 mg/kg body weight of the subject, more preferably between 1.2 and 6 mg/kg body weight of the subject. In an embodiment the low anticoagulant heparin is provided in a dose of 0.9, 1.0, 1.1, 1.2, 1.3, 1.4, 1.5, 1.6, 1.7, 1.8, 1.9, 2.0, 2.2, 2.4, 2.6, 2.8, 3.0, 3.2, 3.4, 3.6, 3.8, 4.0, 4.5, 5.0, 5.0, 5.5, 6.0, 6.5, 7.0, 7.5, 8.0, 8.5, 9.0, 9.5, or 10 mg/kg body weight of the subject.

It is appreciated that the above dosages are based on the specific CRP values and time frames relevant for sepsis, and therefore dosages and treatment times may need to be adapted for other disorders where reducing plasma CRP is relevant. For Example, in case the low anticoagulant heparin is used in the treatment, prevention or amelioration of Alzheimer's disease it is anticipated that treatment should be provided for a much longer period or in repeating intervals. The skilled person will appreciate that in case of long term administration lower dosages may suffice to achieve reduction of plasma CRP levels. The same may apply for treatment, prevention or amelioration of inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, obesity, age related macular degeneration, haemorrhagic stroke, and Parkinson's disease. Thus in a further embodiment the low anticoagulant heparin is administered daily, every two days, every three days, every four days, every five days, every six days, weekly, every two week, every three weeks, every four weeks, monthly, every two months, every three months, every four months, biyearly, or yearly.

In an embodiment the low anticoagulant heparin is administered parenterally. In an embodiment the low anticoagulant heparin is administered intravenously or subcutaneously. Subcutaneous administration may be particularly suitable in case of longer term treatment regimes, which may be relevant for treatment, prevention or amelioration of inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, age related macular degeneration, haemorrhagic stroke, Alzheimer's disease and Parkinson's disease, however other parenteral administration modes are not excluded for these uses.

In an embodiment the level of CRP is reduced to 50% or less in 72 hours. For example the CRP levels have been reduced to levels of 50% compared to CRP levels at diagnosis at 72 hours, preferably 45%, more preferably 40%, 35%, 30%, or 25% or lower. Alternatively the CRP levels have been reduced to levels of 50% compared to CRP levels at diagnosis at 72 hours post diagnosis, preferably at 66 hours, 60 hours, 54 hours, 48 hours, or earlier.

In an embodiment the low anticoagulant heparin is pentasaccharide depleted heparin.

The invention further provides for low anticoagulant heparin for use in the treatment, prevention or amelioration of a diseases in a subject, the disease selected from sepsis, SIRS, septic shock, inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, obesity, age related macular degeneration, haemorrhagic stroke, Alzheimer's disease, and Parkinson's disease, the use comprising administering low anticoagulant heparin to the subject when the plasma CRP levels of the subject exceed 3 mg/L, preferably when the plasma CRP levels of the subject exceed 3, 4, 5, 6, 7, 8, 9, 10, 15, 20, 25, 30, 35, 40, 45, 50, 60, 70, 80, 90 or 100 mg/L. Preferably the use comprises reducing the CRP levels in the plasma of the subject. In an embodiment the low anticoagulant heparin is pentasaccharide depleted heparin.

In a further aspect the invention describes low anticoagulant heparin for use in treating, preventing or ameliorating a disease or disorder in a subject in need thereof, the use comprising lowering plasma CRP levels in the subject. Alternatively the invention provides a method of treating, preventing or ameliorating a disease or disorder in a subject in need thereof, the method comprising lowering plasma CRP levels in the subject by administering low anticoagulant heparin to the subject. In an embodiment the the disease or disorder is selected from sepsis, SIRS, septic shock, inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, age related macular degeneration, haemorrhagic stroke, Alzheimer's disease, and Parkinson's disease. In an embodiment the low anticoagulant heparin is pentasaccharide depleted heparin.

In an embodiment the use comprises measuring the plasma CRP levels in the subject and administering the low anticoagulant heparin when the plasma CRP levels exceed a certain threshold. In an embodiment the threshold is set at 3 mg/L, preferably 4, 5, 6, 7, 8, 9, 10, 15, 20, 25, 30, 35, 40, 45, or 50 mg CRP / L plasma in the subject. In an embodiment the plasma CRP levels in the subject are lowered by administering the low anticoagulant heparin. In an embodiment the low anticoagulant heparin is administered by methods, and/or in dosages or dosage regimes as broadly described above.

### EXAMPLES

### Reference example 1

Typical CRP levels are sourced from Schupp et al. *infra*, patients with sepsis were treated according to standard of care. Patients were divided according to disease status as follows:
(group 1) patients with initial sepsis without progression to septic shock;
(group 2) patients with initial sepsis with progression to septic shock;
(group 3) patients with initial septic shock with clinical improvement to sepsis without shock;
(group 4) patients with initial septic shock without clinical improvement to sepsis without shock.

CRP levels were monitored and plotted in Figures 1. Typically CRP levels initially increase, peak around day 3 and then reduce again to values similar to day 1 at day 5 and to reduced values around day 7.

### Example 2 - Preparation of an AT-column.

The AT-column was prepared according to the package insert of a 5 ml HiTrap column (GE Healthcare^{®}). After washing the isopropanol from the column ~2.5 mg AT in 5 ml coupling buffer was applied to the column. Then, the described procedure to immobilize the protein to the column material and to wash the column was employed (according to the package insert). Finally the column was equilibrated with 140 mM NaCl, 20 mM Tris (pH 7.4).

### Example 3: Separation of UFH into LAM and HAM.

To the column was applied 2 mg unfractionated heparin. LAM was eluted with 140 mM NaCl, 20 mM Tris (pH 7.4) and HAM with 2 M NaCl, 20 mM Tris (pH 7.4). The last buffer was applied in a block gradient. In Fig. 1 an example of the elution pattern is shown.

To obtain a larger amount of LAM, the procedure described in Figure 1 was repeated several times.

To determine whether the LAM was free of HAM two tests were used. Firstly, collected HAM was reapplied to the AT-column and run as described above. No HAM-peak was found. Secondly the effect of LAM on thrombin generation was measured. The reaction mixture (120 µl) contained normal pooled plasma in a 1.5 × dilution, 3 µl LAM or buffer, 4 µM DOPL (60% DOPC, 20% DOPC and 20% DOPE), 5 pM tissue factor (Innovin), 100 mM CaCl2 and 417 µM ZGGR-AMC. The reaction was started with CaCl2 + ZGGR-AMC. Thrombin generation was measured as described by Hemker, H. C., P. Giesen, et al. (2003). Thrombin generation was not inhibited by the added 3 µl HAM. The column fractions containing LAM were collected. The buffer was switched to ammonium bicarbonate (pH 7.8) with Sephadex G-25 medium and the fractions were lyophilized. Dried LAM was weighed and dissolved in phosphate buffered saline to reach the desired concentration.

### Example 4 - intravenous administration of pentasaccharide depleted heparin to septic patients.

### Study Overview

### Brief Summary

Sepsis is a life-threatening organ dysfunction caused by a dysregulated host response to infection. Mortality is high and survivors frequently suffer from long-term sequelae. Extracellular histones have been identified as essential mediators in the pathogenesis of sepsis and septic shock. These toxic molecules are released by damaged cells in response to infection and high extracellular levels can induce tissue injury and multiple organ dysfunction syndrome. Extracellular histones can be neutralized by complexation with the new candidate drug called M6229, a non-anticoagulant heparin, allowing the use of elevated dose levels relative to regular unfractionated heparin. This project aims at the roll-out of a first-in-man clinical study in sepsis patients evaluating the safety, tolerability, pharmacokinetics and pharmacodynamic effects of intravenously administered M6229 in subjects suffering from sepsis.

### Inclusion Criteria:

1. Male or female patients aged ≥ 18 years old.
2. Signed informed consent by patient or legal representative.
3. Diagnosed with sepsis, defined by the Sepsis-3 criteria as a life-threatening organ dysfunction caused by a dysregulated host response to an infection.
   Organ dysfunction is defined by 1 of the following:
   a. Increase in SOFA score of ≥2. i. The baseline SOFA score can be assumed to be zero in patients not known to have pre-existing organ dysfunction.
   b. Acute kidney injury i. Defined as eGFR < 15 mL/min. c. Acute respiratory distress syndrome i. Defined by the Berlin criteria. d. The need of mechanical ventilation. e. Alteration in mental status.
4. The patients have to be included in the study within 72 hours of ICU admission due to sepsis or within 72 hours after sepsis diagnosis on the ICU. M6229 has to be administered within 84 hours after ICU admission due to sepsis or within 84 hours after sepsis diagnosis on the ICU.

### Exclusion Criteria:

1. Subject has an advance directive to withhold life-sustaining treatments.
2. Subject is breastfeeding or intents to get pregnant within 30 days of enrolling into the study.
3. Subject is of childbearing potential and has a positive pregnancy test.
   a. A woman is considered to be of childbearing potential under the age of 60 years, unless surgically sterile.
4. Clinical suspicion or confirmation of a viral hemorrhagic shock syndrome including, but not limited to, dengue fever.
5. Bleeding risk:
   a. Clinical: i. Active bleeding; ii. Head trauma; iii. Intracranial surgery or stroke in the past 3 months; iv. History of intracerebral arteriovenous malformation, cerebral aneurysm or mass lesions of the central nervous system; v. Cerebral haemorrhage; vi. History of a bleeding diatheses; vii. Gastrointestinal bleeding in the past 6 weeks; viii. Presence of an epidural or spinal catheter; ix. Contraindication for IV therapeutic UFH. b. Laboratory: i. Platelet count <50 x109/L; ii. INR >2.0; iii. Baseline aPTT ≥45 seconds prior to enrolment, 1.5x upper limit of normal (ULN).
6. Use of any of the following treatments:
   1. UFH to treat a thrombotic event within 12 hours before infusion;
   2. LMWH within 24 hours before infusion;
   3. Warfarin (if used within 7 days before study entry AND if the INR exceeds 2.0 at enrolment);
   4. Direct oral anticoagulant (DOAC) use 3 days prior to enrollment.
   5. Thrombolytic therapy within 3 previous days;
   6. Use of Ilb/Illa inhibitors within the previous 7 days.
7. Confirmed antiphospholipid syndrome.
8. Known allergy to fish.
9. Cardiopulmonary resuscitation in the previous 7 days.
10. Liver failure defined as Child-Pugh Score Class C.
11. Abnormal liver function (ASAT and/or ALAT > 5 times upper limit of normal (ULN)).
12. Extracorporeal membrane oxygenation (ECMO) support dependent.
13. Pulmonary embolism or clinical suspicion of deep venous thrombosis (DVT).
14. Life expectancy of less than 24 hours.
15. Treating physician refusal.
16. Known adverse reaction to UFH, including heparin induced thrombocytopenia (HIT).
17. Participation in any other investigational drug study or other interventional study with interfering endpoints.
18. Any other clinical condition which, in the opinion of the investigator, would not allow safe completion of the protocol.

### Study plan:

Continuous intravenous infusion of M6229, a low-anticoagulant fraction of heparin. Dose-escalation is based on a modified continual reassessment method (mCRM) including escalation with overdose control (EWOC).

### Outcome Measures

aPTT changes before, during and after infusion of M6229 [Safety and tolerability]; Anti-coagulation effects of M6229 determined by a change in aPTT at different time points during and after infusion of M6229; Up to 72 hours after start infusion
Peak plasma concentration (Cmax) [Pharmacokinetics]; Peak plasma concentration of M6229 in plasma; Up to 72 hours after start infusion.
Steady state concentration (Css) [Pharmacokinetics]; Steady state concentration of M6229 in plasma; Up to 72 hours after start infusion.
Time to peak concentration (Tmax) [Pharmacokinetics]; Time to peak concentration of M6229 in plasma; Up to 72 hours after start infusion.
Area under the plasma concentration versus time curve (AUC) [Pharmacokinetics]; Area under the plasma concentration versus time curve of M6229; Up to 72 hours after start infusion.
Clearance [Pharmacokinetics]; Clearance of M6229; Up to 72 hours after start infusion.
Terminal half-life (t1/2) [Pharmacokinetics]; Terminal half-life is the time required for the plasma concentration of M6229 to fall by 50% during the terminal phase; Up to 72 hours after start infusion.
Volume of distribution (Vd) [Pharmacokinetics]; Volume of distribution of M6229; Up to 72 hours after start infusion.
Histone plasma level changes before, during and after infusion of M6229 [Efficacy]; Change in histone plasma levels before and at different time-points after M6229 administration; Up to 72 hours after start infusion.

### Secondary Outcome Measures

Incidence of excessive anti-coagulation effects [Safety and tolerability]; Excessive anti-coagulation effects are:
Clinical evidence or suspicion of severe non-surgical bleeding, defined as the administration of ≥ 2 units of blood products in 24 hours from start of infusion; aPTT > 90 seconds; Up to 72 hours after start infusion.

Incidence of adverse reactions [Safety and tolerability]; Adverse reactions that are considered definitely and probably related to M6229 as specified in the protocol; Up to 72 hours after start infusion.

Changes in ECG corrected QT interval (QTc) [Safety and tolerability]; Changes in ECGs QTc that are considered definitely and probably related to M6229; Up to 24 hours after start infusion.

Amount of M6229 excreted in urine [Pharmacokinetics]; Urine pharmacokinetic parameters of M6229 (amount of M6229 excreted in urine); Up to 24 hours after start infusion.

Change in plasma levels of D-Dimer before, during and after M6229 administration [Efficacy]; Change in plasma levels of biomarkers of inflammation, coagulation and fibrinolysis (e.g. D-dimer, IL-6, IL-8) before and at different time-points after M6229 administration; Up to 72 hours after start infusion.

Change in plasma levels of interleukins before, during and after M6229 administration [Efficacy]; Change in plasma levels of biomarkers of inflammation, coagulation and fibrinolysis (e.g. D-dimer, IL-6, IL-8) before and at different time-points after M6229 administration; Up to 72 hours after start infusion

Correlation of histone plasma levels and abovementioned biomarkers with M6229 plasma levels (PK/PD) [Efficacy]; Besides histone plasma levels, the investigators will also measure other biomarkers of inflammation, coagulation and fibrinolysis (e.g. D-dimer, IL-6, IL-8); Up to 72 hours after start infusion

Severity of organ dysfunction based on Sequential Organ Failure Assessment (SOFA) score [Efficacy]; SOFA scores will be reported. Moreover, the investigators will compare these data with historic controls. For this, data will be used from a subset of patients included in a previously conducted study conducted in two tertiary teaching hospitals in the Netherlands named "Molecular Diagnosis and Risk Stratification of Sepsis" (MARS) study. The MARS study was a prospective observational study performed between January 2011 and January 2014 in the ICUs of the Amsterdam UMC, location AMC and UMC Utrecht; 30 days.

Time on mechanical ventilation [Efficacy]; Ventilator free-days and time on mechanical ventilation. Data will be compared with historic controls from the MARS cohort; 30 days.

Time on renal replacement therapy [Efficacy]; Renal replacement therapy free-days and time on renal replacement therapy. Data will be compared with historic controls from the MARS cohort; 30 days.

Time on vasopression therapy [Efficacy]; Vasopressor free-days and time on vasopressors. Data will be compared with historic controls from the MARS cohort; 30 days.

Length of stay [Efficacy]; ICU and hospital length of stays. Data will be compared with historic controls from the MARS cohort; 30 days

Mortality rate [Efficacy]; ICU and hospital mortality. Data will be compared with historic controls from the MARS cohort; 30 days.

Pentasaccharide depleted heparin was prepared as indicated above. Patients diagnosed with sepsis were administered pentasaccharide depleted heparin in a dosage as indicated below:
Dose level 1 - patients 2 and 3: 0.15 mg pentasaccharide depleted heparin per kilogram of body weight per hour;
Dose level 2 - patients 4 and 5: 0.45 mg pentasaccharide depleted heparin per kilogram of body weight per hour;
Dose level 3 - patients 6-11: 0.9 mg pentasaccharide depleted heparin per kilogram of body weight per hour;
dosages were assigned to patients arbitrarily.

Administration was performed intravenously by infusion for a duration of 6 hours after diagnosis. Blood samples were taken at the following time points in hours: T=0 (start of pentasaccharide depleted heparin administration), T=3, T=6 (end of pentasaccharide depleted heparin administration), T=24, T=48, T=72, and CRP values are determined, among others. CRP values for each time point are plotted per patient in Figure 2 and combined in Figure 3. The values indicated mg CRP detected per mL plasma.

### Cited references

Schupp et al. Irish Journal of Medical Science (2024) 193:457-468.
Ang et al. Aliment Pharmacol Ther 2000; 14: 1015-1022.
Ashoor et al., Korean J Anesthesiol 2020;73(6):509-517.
Bhattacharya et al. (2023) Front. Immunol. 14:1238411.
Casu, B. et al. Biochem J 197(3) (1981) 599-609.
Casu, B et al. (1989). "Structure of heparin and heparin fragments." Ann N Y Acad Sci 556: 1-17.
Folwaczny et al., THE AMERICAN JOURNAL OF GASTROENTEROLOGY Vol. 94, No. 6, 1999, 1551-1555.
Hemker, H. C., P. Giesen, et al. (2003). "Calibrated automated thrombin generation measurement in clotting plasma." Pathophysiol Haemost Thromb 33(1): 4-15
Jaimes et al. Crit Care Med. 2009 Apr;37(4):1185-96.
Jin et al. J Dig Dis. 2018 Dec;19(12):766-772.
Lu et al., The American Journal of Emergency Medicine, Volume 36, Issue 10, 2018, Pages 1789-1795.
Povoa et al. Intensive Care Med (1998) 24: 1052-1056.
Qiu et al. J Dig Dis. 2019;20:512-522.
Raman et al., Methods Mol Biol 2015:1229:31-6.
Singer et al. JAMA. 2016 February 23; 315(8): 801-810.
Wang et la. Carbohydrate Polymers 295 (2022) 119818.

## Claims

1. Low anticoagulant heparin for use in the treatment or prevention of sepsis, SIRS or septic shock in a subject,
wherein the pentasaccharide-depleted heparin is administered to the subject in a dose of at least 0.15 mg/(kg*hr) body weight of the subject.

2. Low anticoagulant heparin for use according to claim 1, wherein the low anticoagulant heparin is administered to the subject in a dose of at least 0.45 mg/(kg*hr) body weight of the subject.

3. Low anticoagulant heparin for use according to claim 1, wherein the low anticoagulant heparin is administered to the subject in a dose of at least 0.9 mg/(kg*hr) body weight of the subject.

4. Low anticoagulant heparin for use according to any one of the preceding claims wherein the low anticoagulant heparin is administered parenterally, preferably wherein the low anticoagulant heparin is administered intravenously or subcutaneously.

5. Low anticoagulant heparin for use according to any one of the preceding claims wherein the low anticoagulant heparin is administered over a period of at least 2 hours, preferably at least 4 hours more preferably at least 5 hours, or wherein the low anticoagulant heparin is administered over a period of at least 48 hours, preferably at least 60, at least 72, at least 96, more preferably at least 120 hours or longer.

6. Low anticoagulant heparin for use according to any one of the preceding claims wherein the use comprises reducing the amount of C reactive protein (CRP) in the subject.

7. Low anticoagulant heparin for use according to claim 6, wherein the level of CRP is reduced to 50% or less in 72 hours.

8. Low anticoagulant heparin for use in the treatment or prevention of a disease, the use comprising reducing C-reactive protein (CRP) in the patient.

9. Low anticoagulant heparin for use according to claim 8, wherein the disease is selected from sepsis, SIRS, septic shock, inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, obesity, age related macular degeneration, haemorrhagic stroke, Alzheimer's disease, and Parkinson's disease

10. Low anticoagulant heparin for use according to claim 8 or 9, wherein the low anticoagulant heparin is administered to the subject in a dose of at least 0.15 mg/(kg*hr) body weight of the subject, preferably in a dose of at least 0.45 mg/(kg*hr) body weight of the subject, more preferably in a dose of at least 0.9 mg/(kg*hr) body weight of the subject.

11. Low anticoagulant heparin for use according to any one of claims 8 to 10, wherein the low anticoagulant heparin is administered parenterally preferably wherein the low anticoagulant heparin is administered intravenously or subcutaneously.

12. Low anticoagulant heparin for use according to any one of claims 8 to 11, wherein the low anticoagulant heparin is administered over a period of at least 2 hours, preferably at least 4 hours more preferably at least 5 hours, or wherein the low anticoagulant heparin is administered over a period of at least 48 hours, preferably at least 60, at least 72, at least 96, more preferably at least 120 hours or longer.

13. Low anticoagulant heparin for use according to any one of claims 8 to 12, wherein the level of CRP is reduced to 50% or less in 72 hours.

14. Low anticoagulant heparin for use in the treatment, prevention or amelioration of a diseases in a subject, the disease selected from sepsis, SIRS, septic shock, inflammatory bowel disease (IBD), Chronic obstructive pulmonary disease (COPD), pancreatitis, abdominal aortic aneurism, atherosclerosis, (rheumatoid) arthritis, cardiovascular disease, type 2 diabetes mellitus, obesity, age related macular degeneration, haemorrhagic stroke, Alzheimer's disease, and Parkinson's disease, the use comprising administering low anticoagulant heparin to the subject when the plasma CRP levels of the subject exceed 3 mg/L

15. Low anticoagulant heparin for use in treating, preventing or ameliorating a disease or disorder in a subject in need thereof, the use comprising lowering plasma CRP levels in the subject.
